# EUROPEAN PATENT APPLICATION

(11) **EP 3 584 259 A1**
(43) Date of publication of application: **25.12.2019**
(21) Application number: 18202727.6
(22) Date of filing: 17.10.2013
(51) Int. Cl.: C07K 16/28, C07K 16/32, A61K 39/395, A61P 35/00

(54) **COMBINATIONS OF EPIDERMAL GROWTH FACTOR RECEPTOR TARGETING ANTIBODIES FOR TREATING CANCER**

(30) Priority: 24.10.2012 US 201261717733 P
(62) Divisional of application: 13786318.9
(71) Applicant: Yeda Research and Development Co., Ltd., 7610002 Rehovot (IL)
(72) Inventor: YARDEN, Yosef, 7630921 Rehovot (IL); SELA, Michael, 7610002 Rehovot (IL); MARON, Ruth, 7610002 Rehovot (IL); FERRARO, Daniela Aleida, 7610002 Rehovot (IL)
(74) Representative: Dennemeyer & Associates S.A.

(57) **Abstract**

The present invention relates to the use of an anti-EGFR antibody combination for treating pancreatic cancer, wherein the combination comprises: (i) a first anti-EGFR antibody comprising the CDR sequences of anti EGFR 565 (CNCM Deposit Number I-4262), wherein said first anti-EGFR antibody binds the same EGFR epitope as said anti EGFR 565; and (ii) a second anti-EGFR antibody comprising the CDR sequences of C225/Cetuximab, wherein said second anti-EGFR antibody binds the same EGFR epitope as said C225/Cetuximab.

## Description

### FIELD AND BACKGROUND OF THE INVENTION

The present invention, in some embodiments thereof, relates to antibody combinations which target the epidermal growth factor receptor for the treatment of cancer and more specifically, breast cancer.

Growth factors and their transmembrane receptor tyrosine kinases (RTKs) play critical roles in tumor progression. One remarkable example entails a large family of growth factors, all sharing an epidermal growth factor (EGF) motif, and their respective RTKs of the ERBB/HER family. Consistent with essential roles in tumor progression, strategies able to interfere with ERBB functions, such as monoclonal antibodies (mAbs) and tyrosine kinase inhibitors (TKIs), have yielded in the last decade several oncology drugs. For example, two genetically engineered mAbs to EGFR, cetuximab and panitumumab, are approved for treatment of colorectal cancer. Unlike the well-understood mode of action of TKIs, the mechanisms underlying therapeutic activities of mAbs are less understood. In general, potential mechanisms can be divided into immune-mediated cell killing, such as antibody-dependent cellular cytotoxicity (ADCC), and diverse neutralizing effects, such as inhibition of ligand binding, prevention of receptor dimerization, and induction of receptor internalization.

Early animal studies that tested a set of mAbs to the rodent form of ERBB2/HER2 indicated that individual mAbs causes partial tumor eradication, whereas the administration of certain mixtures of antibodies resulted in synergistic effects. Similar effects on the human HER2 protein were later confirmed. *In vitro,* the more effective mAb mixture was also more effective than the single mAbs in inducing receptor degradation and ADCC. Synergistic anti-tumor effects were confirmed, as well as associated with receptor degradation, using another set of mAbs to HER2. Importantly, a mixture of two mAbs to HER2, trastuzumab and pertuzumab, in combination with chemotherapy, significantly prolonged progression-free survival of breast cancer patients whose tumors overexpress HER2. Similar to anti-HER2 mAbs, cetuximab induces down-regulation of EGFR, and this effect appears important for growth inhibition. Experiments that employed a radiolabeled cetuximab confirmed endocytosis of the mAb, but the internalized mAb recycled more effectively than internalized EGF.

Similar to the synergistic internalizing effects of combinations of HER2-directed mAbs, certain pairs of anti-EGFR antibodies were found to accelerate receptor endocytosis and degradation [Friedman et al., Proceedings of the National Academy of Sciences of the United States of America 102(6):1915-1920]. To enhance endocytosis, the mAbs must engage non-overlapping antigenic epitopes of EGFR. Another study showed that highly potent mAb combinations reduced surface receptor levels through a mechanism consistent with mAb-mediated inhibition of EGFR recycling [Spangler et al., Proceedings of the National Academy of Sciences of the United States of America 107(30):13252-13257].

U.S. Patent Nos. 7,498,142 and 7,939,072 teach combinations of anti-EGFR antibodies 111 and 565 for the treatment of cancer.

International Patent Application No. IL2012/050176 teaches combinations of anti-EGFR antibodies 111 and 565 for the treatment of pancreatic cancer.

### SUMMARY OF THE INVENTION

According to an aspect of some embodiments of the present invention there is provided a method of treating triple negative breast cancer in a subject in need thereof, comprising administering to the subject a therapeutically effective amount of a combination of at least two anti epidermal growth factor receptor (EGF-R) antibodies exhibiting binding to different epitopes on the EGF-R, thereby treating triple negative breast cancer.

According to an aspect of some embodiments of the present invention there is provided a method of treating cancer in a subject comprising administering to the subject a therapeutically effective amount of a combination of at least two anti EGF-R antibodies exhibiting binding to different epitopes on the EGF-R, wherein the combination is selected from the group consisting of:
(i) an anti-EGFR antibody comprising the CDR sequences of anti EGFR 565 (CNCM Deposit Number I-4262); and an anti-EGFR antibody comprising the CDR sequences of C225/Cetuximab™;
(ii) an anti-EGFR antibody comprising the CDR sequences of anti EGFR 111 (CNCM Deposit Number I-4261); and an anti-EGFR antibody comprising the CDR sequences of C225/Cetuximab™;
(iii) an anti-EGFR antibody comprising the CDR sequences of anti EGFR 565 (CNCM Deposit Number 1-4262); and an anti-EGFR antibody comprising the CDR sequences of panitumumab™; and
(iv) an anti-EGFR antibody comprising the CDR sequences of anti EGFR 111 (CNCM Deposit Number I-4261); and an anti-EGFR antibody comprising the CDR sequences of panitumumab™.

According to an aspect of some embodiments of the present invention there is provided an article-of-manufacture comprising a packaging material identified for treating triple negative breast cancer, packaging two anti EGF-R antibodies exhibiting binding to different epitopes on the EGF-R.

According to an aspect of some embodiments of the present invention there is provided a pharmaceutical composition comprising as active ingredients two anti EGF-R antibodies exhibiting binding to different epitopes on the EGF-R and a pharmaceutically acceptable carrier or diluent.

According to an aspect of some embodiments of the present invention there is provided an article-of-manufacture comprising a packaging material identified for treating cancer, packaging:
(i) an anti-EGFR antibody comprising the CDR sequences of anti EGFR 565 (CNCM Deposit Number 1-4262); and an anti-EGFR antibody comprising the CDR sequences of C225/Cetuximab™; or
(ii) an anti-EGFR antibody comprising the CDR sequences of anti EGFR 111 (CNCM Deposit Number I-4261); and an anti-EGFR antibody comprising the CDR sequences of C225/Cetuximab™; or
(iii) an anti-EGFR antibody comprising the CDR sequences of anti EGFR 565 (CNCM Deposit Number 1-4262); and an anti-EGFR antibody comprising the CDR sequences of panitumumab™; or
(iv) an anti-EGFR antibody comprising the CDR sequences of anti EGFR 111 (CNCM Deposit Number I-4261); and an anti-EGFR antibody comprising the CDR sequences of panitumumab™.

According to an aspect of some embodiments of the present invention there is provided a pharmaceutical composition comprising active ingredients selected from the group consisting of:
(i) an anti-EGFR antibody comprising the CDR sequences of anti EGFR 565 (CNCM Deposit Number 1-4262); and an anti-EGFR antibody comprising the CDR sequences of C225/Cetuximab™; or
(ii) an anti-EGFR antibody comprising the CDR sequences of anti EGFR 111 (CNCM Deposit Number I-4261); and an anti-EGFR antibody comprising the CDR sequences of C225/Cetuximab™; or
(iii) an anti-EGFR antibody comprising the CDR sequences of anti EGFR 565 (CNCM Deposit Number 1-4262); and an anti-EGFR antibody comprising the CDR sequences of panitumumab™; or
(iv) an anti-EGFR antibody comprising the CDR sequences of anti EGFR 111 (CNCM Deposit Number 1-4261); and an anti-EGFR antibody comprising the CDR sequences of panitumumab™,
and a pharmaceutically acceptable carrier or diluent.

According to an aspect of some embodiments of the present invention there is provided an anti-EGFR antibody combination comprising:
(i) an anti-EGFR antibody comprising the CDR sequences of anti EGFR 565 (CNCM Deposit Number 1-4262); and an anti-EGFR antibody comprising the CDR sequences of C225/Cetuximab™; or
(ii) an anti-EGFR antibody comprising the CDR sequences of anti EGFR 111 (CNCM Deposit Number I-4261); and an anti-EGFR antibody comprising the CDR sequences of C225/Cetuximab™; or
(iii) an anti-EGFR antibody comprising the CDR sequences of anti EGFR 565 (CNCM Deposit Number 1-4262); and an anti-EGFR antibody comprising the CDR sequences of panitumumab™; or
(iv) an anti-EGFR antibody comprising the CDR sequences of anti EGFR 111 (CNCM Deposit Number I-4261); and an anti-EGFR antibody comprising the CDR sequences of panitumumab™ for use in treating cancer.

According to some embodiments of the invention, the combination is selected from the group consisting of:
(i) an anti-EGFR antibody comprising the CDR sequences of anti EGFR 565 (CNCM Deposit Number 1-4262); and an anti-EGFR antibody comprising the CDR sequences of C225/Cetuximab™;
(ii) an anti-EGFR antibody comprising the CDR sequences of anti EGFR 111 (CNCM Deposit Number I-4261); and an anti-EGFR antibody comprising the CDR sequences of C225/Cetuximab™;
(iii) an anti-EGFR antibody comprising the CDR sequences of anti EGFR 565 (CNCM Deposit Number 1-4262); and an anti-EGFR antibody comprising the CDR sequences of panitumumab™;
(iv) an anti-EGFR antibody comprising the CDR sequences of anti EGFR 111 (CNCM Deposit Number I-4261); and an anti-EGFR antibody comprising the CDR sequences of panitumumab™.

According to some embodiments of the invention, the cancer is breast cancer. According to some embodiments of the invention, the method further comprises subjecting the subject to a therapy selected from the group consisting of a radiotherapy and a chemotherapy.

According to some embodiments of the invention, the administering comprises multiple administrations.

According to some embodiments of the invention, the multiple administrations comprise weekly administrations.

According to some embodiments of the invention, the active ingredients are in a co-formulation.

According to some embodiments of the invention, the active ingredients are in separate formulations.

According to some embodiments of the invention, the two anti epidermal growth factor receptor (EGF-R) antibodies exhibiting binding to different epitopes on the EGF-R are for use in treating triple negative breast cancer.

According to some embodiments of the invention, the packaging material comprises at least two containers for separately packaging the antibodies.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying images. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIGs. 1A-F illustrate specific combinations of anti-EGFR mAbs that enhance receptor downregulation and degradation. (A) MDA-MB-468 cells were incubated for 45 min at 4°C with increasing concentrations of growth factors or mAbs. Thereafter, Texas Red-EGF (300 nM) was added for additional 45 minutes. Fluorescence intensity was determined after washing, using a microplate reader. The results represent averages (±S.D.) from three experiments. (B and C) Serum starved MDA-MB-468 cells were incubated at 4°C with the indicated mAbs (or with EGF). Cetuximab (*B; ctx*) or panitumumab (*C; pan*), each at 1µg/ml, were added 15 minutes later and incubated for 45 minutes. The cells were then washed and incubated for 60 minutes at 4°C with HRP-labeled anti human IgG. Light absorbance (415 nm) was determined using an ELISA microplate reader after washing and incubating for 15 minutes with 2, 2' Azino-bis (3-ethylbenzothiazoline-6-sulfonic acid). (D) HeLa cells were incubated for 12 hours with the indicated mAbs (20 µg/ml), or for 60 minutes with EGF (10 ng/ml). Bound ligands were acid-stripped, and surface EGFR was measured by FACS analysis. Values are the average±S.D. of triplicates. (*E*) HeLa cells were treated for 12 hours with the indicated anti-EGFR mAbs (20 µg/ml), or for 60 minutes with EGF (10 ng/ml). Afterwards, whole cell extracts were subjected to immunoblotting (IB) with the indicated antibodies. (*F*) HeLa cells were incubated for the indicated intervals with mAbs (20 µg/ml), or with EGF (10 ng/ml), and lysates were probed with the indicated antibodies.
FIGs. 2A-C illustrate that anti-EGFR antibodies enhance receptor ubiquitination and degradation. (*A*) Serum-starved HeLa cells were incubated with mAbs (10 µg/ml), their combination (each at 5 µg/ml), or with EGF (10 ng/ml), and lysates analyzed using immunoprecipitation (IP) and immunoblotting (IB). (B) HeLa cells transfected with a plasmid encoding a MYC peptide tagged CBL, or an empty vector, were serum-starved and treated for 3h with the indicated mAbs (20 µg/ml) or their combination (each at 10 µg/ml). Alternatively, cells were treated for 10 minutes with EGF (10 ng/ml). Lysates were probed as indicated. (C) HeLa cells that were pre-incubated (12h) with bortezomib (2 µM) or bafilomycin (10 nM), were incubated (60 min) with EGF (10 ng/ml) or for 6h with the indicated combination of mAbs (each at 10 µg/ml). Lysates were subjected to immunoblotting (IB) and signal quantification.
FIGs. 3A-C illustrate that a combination of anti-EGFR antibodies enhances receptor internalization and impairs recycling. (*A* and *B*) Serum-starved HeLa cells were incubated with EGF (10 ng/ml; 30 min), or mAbs (20 µg/ml total; 4h). Thereafter, cells were permeabilized and EGFR was localized using flow cytometry. EGFR distribution between the plasma membrane (scored as 1) and the cell's center (scored as 0) was assessed. (*C*) HeLa cells were treated with EGF (10 ng/ml; 30 min) or mAbs (10 µg/ml total; 4h). Following fixation, permeabilization, and incubation with anti-EGFR and anti-Rabll antibodies, cells were incubated with fluorescent secondary antibodies, and images were acquired with a confocal fluorescent microscope. Co-localization of two markers appears as yellow (*Merge*)*.*
FIGs. 4A-C illustrate that a combination of anti-EGFR mAbs downregulates a dimerization-defective mutant of EGFR but cannot trigger downstream signaling. (*A*) Serum-starved HeLa cells were incubated with EGF (10 ng/ml; 1h) or with anti-EGFR mAbs (20 µg/ml total; 12h) and lysates probed as indicated. (B) HeLa cells were transfected with a plasmid encoding a dimerization-defective mutant of EGFR (ΔCR-EGFR-YFP). Forty-eight hours later, serum starved cells were treated with EGF (10 ng/ml) or with mAbs (20 µg/ml total), prior to lysate immunoblotting. (*C*) HeLa cells were pretreated for 4 hours with a selective EGFR kinase inhibitor (AG1478; 10 µM). Subsequently, cells were washed and treated with EGF (10 ng/ml), or with a combination of mAbs (each at 10 µg/ml). Lysates were probed with the indicated antibodies.
FIGs. 5A-D illustrate that a combination of antibodies downregulates EGFR and inhibits invasion of TNBC cells. (*A*) Whole extracts were prepared from the indicated cell lines after treatment with EGF (10 ng/ml; 1h) or with the mAbs (20µg/ml total; 6h). Lysates were immunoblotted as indicated. (*B*) BT-549 cells were treated for 48 hours with mAbs (20 µg/ml total) and lysates immunoblotted for EGFR and ERK2. (*C* and *D*) BT-549 cells were treated with mAbs as in B. Thereafter, cells were plated in the upper compartment of invasion chambers. The lower compartments were filled with the respective mAb-containing media. Eighteen hours later, the filters were removed, fixed, permeabilized and stained with methyl violet (0.3%). Cells growing on the upper side of the filter were removed and cells on the bottom side were photographed and quantified.
FIGs. 6A-C illustrate that a combination of anti-EGFR mAbs interferes with EGF-dependent proliferation and with in vivo growth of TNBC cells. (A) HCC70 cells (3,000 cells per well) were seeded in 12-well plates and allowed to adhere overnight. Afterwards, cells were incubated for 5 days in serum-free medium containing mAbs (20 µg/ml total) in the absence or presence of EGF (10 ng/ml). Following fixation and staining with Giemsa (0.2% in saline), cells were photographed and quantified. Shown are the results of one out of three experiments. (B and C) Groups of five CDl/nude mice were injected subcutaneously with 7 x 10⁶ HCC70 cells. Antibodies (total: 160 µg/animal/injection) were weekly injected intraperitoneally, once tumors became palpable. Tumor volumes were assessed once per week. ** indicates *p* < 0.01 by two-way analysis of variance with Bonferroni's multiple comparison post-tests. The average tumor size in each group ± SEM is presented.
FIGs. 7A-B are analyses of mutual antibody competition. HeLa cells were treated for 60 minutes at 4°C with increasing concentrations mAbs 565 and panitumumab (*A*), or with the mAbs cetuximab and panitumumab (*B*). Afterwards, a radiolabeled panitumumab was added (10 nmol/L), and cells were incubated for additional 15 minutes on ice. Thereafter, cell-bound radioactivity was determined. Averages of triplicates and S.D. values (bars) are presented.
FIGs. 8A-B illustrate the identification of specific effectors of the mAb-induced receptor degradation pathway using a screen of siRNA oligonucleotides. (A) HeLa cells were seeded in 24-well plates and then transfected with a mixture of four siRNA oligonucleotides (50 nM), each targeting a specific gene involved in endocytosis. After 48 hours, cells were starved for 12 hours and then incubated for 6 hours with a combination of mAbs 111 and 565 (each at 10 µg/ml). The cells were then lysed and whole cell extracts were probed with an anti-EGFR antibody, and with anti-GAPDH antibody as a loading control. Shown is a collection of genes whose knockdown inhibited mAb-induced degradation of EGFR, genes that exerted minimal or no effects, and several control assays. (*B*) The ability of selected siRNA mixtures to block mAb-induced degradation of EGFR was scored relative to the control by densitometry.
FIG. 9 illustrates that a combination of mAbs can induce EGFR internalization. HeLa cells were serum starved for 12 hours, and thereafter incubated for 30 minutes with EGF (10 ng/ml). Alternatively, cells were incubated for 4 hours with panitumumab and mAb111, either separately or in combination (20 µg/ml). Afterwards, cells were permeabilized, and EGFR was detected using a primary and a secondary, ALEXA-488 labeled, antibody. The localization of fluorescent signals was assessed using the ImageStreamX instrument.
FIGs. 10A-D illustrate that suppression of EGFR inhibit invasion of TNBC cells. (A) Whole cell extracts were prepared from the indicated breast cancer cell lines. As control, we used normal mammary cells (MCF10A) and the EGFR-overexpressing A-431 human epidermoid carcinoma cells. Whole cell extracts were subjected to immunoprecipitation (IP) and/or immunoblotting (IB) with the indicated antibodies. (B) BT-549 cells were transfected with a pool of four siRNA oligonucleotides targeting EGFR, or with control oligonucleotides. Forty-eight hours later, cells were lysed and whole cell extracts were immunoblotted with anti-EGFR antibodies. (*C* and *D*) BT-549 cells were transfected with a pool of four siRNA oligonucleotides targeting EGFR, or with control oligonucleotides. Forty-eight hours later, the cells were seeded in the upper compartment of migration or invasion chambers. The lower compartment of each chamber was filled with serum-containing medium. After 18 hours, cells in the lower side of the filter were fixed, permeabilized, stained and photographed. Areas covered by cells were quantified and presented in a histogram.
FIGs. 11A-B illustrate that a combination of anti-EGFR mAbs induces cell cycle arrest, with no detectable apoptosis. (A) HS578T human TNBC cells were incubated with the mAbs cetuximab and 565, either alone (at 20 µg/ml) or in combination (each at 10 µg/ml), for either two or four days, as indicated. Alternatively, cells were incubated with puromycin (2 ng/ml; 12h). Subsequently, cells were fixed for 12 hours in ice-cold methanol and re-suspended in saline containing propidium iodide (0.02 mg/ml). The incorporation of propidium iodide into DNA, which reflects cell cycle distribution, was measured using flow cytometry. (*B*) HS578T cells were treated for the indicated time intervals with cetuximab or mAb 565 as in A. As positive control, the cells were treated for 12 hours with puromycin (2 ng/ml). Whole cell lysates were immunoblotted (IB) with antibodies specific to the cleaved, active form of caspase-3. Likewise, antibodies directed to both the intact and the cleaved forms of caspase 3 were also used.
FIGs. 12A-B illustrate that a mixture of antibodies to EGFR inhibits tumorigenic growth of human TNBC cells in animals. Groups of five CD1/nude mice were injected subcutaneously with 7 x 10⁶ HCC70 cells. Once tumors became palpable, the mAbs cetuximab, panitumumab and 111 were weekly injected intraperitoneally, either singly (160 µg/animal/injection) or in combination (each mAb at 80 µg/animal/injection). Tumor volumes were assessed once per week. ** indicates *p* < 0.01 by two-way analysis of variance with Bonferroni's multiple comparison post-tests. The average tumor size in each group (± SEM) is presented.

### DESCRIPTION OF SPECIFIC EMBODIMENTS OF THE INVENTION

The present invention, in some embodiments thereof, relates to antibody combinations which target the epidermal growth factor receptor (EGFR) for the treatment of cancer and more specifically, breast cancer.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

Breast tumors lacking expression of HER2, the estrogen- and the progesterone receptors ('triple negative'; TNBC) are more aggressive than other disease subtypes, and no molecular targeted agents are currently available for their treatment. The present inventors addressed the relevance of a combination of non-competitive monoclonal antibodies which target EGFR for the treatment of TNBC. Unlike a combination of the clinically approved monoclonal antibodies, cetuximab and panitumumab, which displaced each other and displayed no cooperative effects, several other combinations resulted in enhanced inhibition of TNBC's cell growth (e.g., 111/panitumumab and 111/cetuximab). Such effects were demonstrated both *in vitro* (Figures 5A-D) and in animals (Figures 6A-C).

Thus, according to one aspect of the present invention there is provided a method of treating cancer in a subject comprising administering a therapeutically effective amount of a combination of at least two anti epidermal growth factor receptor (EGF-R) antibodies exhibiting binding to different epitopes on the EGF-R.

According to an embodiment of this aspect of the present invention, the antibodies comprise anti-tumor activity.

As used herein the phrase "anti-tumor activity" refers to prevention of tumor formation and/or reduction of tumor size (e.g., volume) and/or metastasis potential.

The combination of antibodies described herein has combined improved anti tumor activity. As used herein the phrase "combined improved anti-tumor activity" refers to at least additive but preferably synergistically improved anti-tumor activity.

As used herein the term "synergy" refers a total affect that is greater than the sum of the individual contribution of each antibody.

As used herein "EGF-R" refers to a receptor tyrosine kinase (RTK) of the epidermal growth factor receptor family, EGFR_HUMAN, P00533, also referred to as HER1, mENA and ErbB-1.

Antibodies of this aspect of the present invention may bind the EGF-R with similar or different affinities. According to another preferred embodiment of this aspect of the present invention, the antibodies bind EGF-R with a minimal affinity of at least 1 µM, 200 nM, 100 nM, 1 nM or higher.

Antibodies of this aspect of the present invention can be selected from pre-existing antibodies (e.g., publicly available hybridomas or recombinant antibody libraries, further described hereinbelow) or from newly generated antibodies produced according to methods which are well-known in the art and are further described herein below.

Antibodies and methods of generating same are described at length in the following sections.

The term "antibody" as used in this invention includes intact molecules as well as functional fragments thereof, such as Fab, F(ab')2, and Fv. These functional antibody fragments are defined as follows: (1) Fab, the fragment which contains a monovalent antigen-binding fragment of an antibody molecule, can be produced by digestion of whole antibody with the enzyme papain to yield an intact light chain and a portion of one heavy chain; (2) Fab', the fragment of an antibody molecule that can be obtained by treating whole antibody with pepsin, followed by reduction, to yield an intact light chain and a portion of the heavy chain; two Fab' fragments are obtained per antibody molecule; (3) (Fab')2, the fragment of the antibody that can be obtained by treating whole antibody with the enzyme pepsin without subsequent reduction; F(ab')2 is a dimer of two Fab' fragments held together by two disulfide bonds; (4) Fv, defined as a genetically engineered fragment containing the variable region of the light chain and the variable region of the heavy chain expressed as two chains; and (5) Single chain antibody ("SCA"), a genetically engineered molecule containing the variable region of the light chain and the variable region of the heavy chain, linked by a suitable polypeptide linker as a genetically fused single chain molecule.

Methods of producing polyclonal and monoclonal antibodies as well as fragments thereof are well known in the art (See for example, Harlow and Lane, Antibodies: A Laboratory Manual, Cold Spring Harbor Laboratory, New York, 1988, incorporated herein by reference).

Antibody fragments according to the present invention can be prepared by proteolytic hydrolysis of the antibody or by expression in E. coli or mammalian cells (e.g. Chinese hamster ovary cell culture or other protein expression systems) of DNA encoding the fragment. Antibody fragments can be obtained by pepsin or papain digestion of whole antibodies by conventional methods. For example, antibody fragments can be produced by enzymatic cleavage of antibodies with pepsin to provide a 5S fragment denoted F(ab')2. This fragment can be further cleaved using a thiol reducing agent, and optionally a blocking group for the sulfhydryl groups resulting from cleavage of disulfide linkages, to produce 3.5S Fab' monovalent fragments. Alternatively, an enzymatic cleavage using pepsin produces two monovalent Fab' fragments and an Fc fragment directly. These methods are described, for example, by Goldenberg, U.S. Pat. Nos. 4,036,945 and 4,331,647, and references contained therein, which patents are hereby incorporated by reference in their entirety. See also Porter, R. R. [Biochem. J. 73: 119-126 (1959)]. Other methods of cleaving antibodies, such as separation of heavy chains to form monovalent light-heavy chain fragments, further cleavage of fragments, or other enzymatic, chemical, or genetic techniques may also be used, so long as the fragments bind to the antigen that is recognized by the intact antibody.

Fv fragments comprise an association of VH and VL chains. This association may be noncovalent, as described in Inbar et al. [Proc. Nat'l Acad. Sci. USA 69:2659-62 (19720]. Alternatively, the variable chains can be linked by an intermolecular disulfide bond or cross-linked by chemicals such as glutaraldehyde. Preferably, the Fv fragments comprise VH and VL chains connected by a peptide linker. These single-chain antigen binding proteins (sFv) are prepared by constructing a structural gene comprising DNA sequences encoding the VH and VL domains connected by an oligonucleotide. The structural gene is inserted into an expression vector, which is subsequently introduced into a host cell such as E. coli. The recombinant host cells synthesize a single polypeptide chain with a linker peptide bridging the two V domains. Methods for producing sFvs are described, for example, by [Whitlow and Filpula, Methods 2: 97-105 (1991); Bird et al., Science 242:423-426 (1988); Pack et al., Bio/Technology 11:1271-77 (1993); and U.S. Pat. No. 4,946,778, which is hereby incorporated by reference in its entirety.

Another form of an antibody fragment is a peptide coding for a single complementarity-determining region (CDR). CDR peptides ("minimal recognition units") can be obtained by constructing genes encoding the CDR of an antibody of interest. Such genes are prepared, for example, by using the polymerase chain reaction to synthesize the variable region from RNA of antibody-producing cells. See, for example, Larrick and Fry [Methods, 2: 106-10 (1991)].

Humanized forms of non-human (e.g., murine) antibodies are chimeric molecules of immunoglobulins, immunoglobulin chains or fragments thereof (such as Fv, Fab, Fab', F(ab').sub.2 or other antigen-binding subsequences of antibodies) which contain minimal sequence derived from non-human immunoglobulin. Humanized antibodies include human immunoglobulins (recipient antibody) in which residues form a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as import residues, which are typically taken from an import variable domain. Humanization can be essentially performed following the method of Winter and co-workers [Jones et al., Nature, 321:522-525 (1986); Riechmann et al., Nature 332:323-327 (1988); Verhoeyen et al., Science, 239:1534-1536 (1988)], by substituting rodent CDRs or CDR sequences for the corresponding sequences of a human antibody. Accordingly, such humanized antibodies are chimeric antibodies (U.S. Pat. No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human antibodies can be made by introduction of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Pat. Nos. 5,545,807; 5,545,806; 5,569,825; 5,625,126; 5,633,425; 5,661,016, and in the following scientific publications: Marks et al., Bio/Technology 10,: 779-783 (1992); Lonberg et al., Nature 368: 856-859 (1994); Morrison, Nature 368 812-13 (1994); Fishwild et al., Nature Biotechnology 14, 845-51 (1996); Neuberger, Nature Biotechnology 14: 826 (1996); and Lonberg and Huszar, Intern. Rev. Immunol. 13, 65-93 (1995).

As used herein, the term "epitope" refers to any antigenic determinant on an antigen to which the paratope of an antibody binds.

Epitopic determinants usually consist of chemically active surface groupings of molecules such as amino acids or carbohydrate side chains and usually have specific three dimensional structural characteristics, as well as specific charge characteristics.

As mentioned the antibodies of the present invention bind different epitopes on EGFR. The epitopes may be conformational or not and may be overlapping or not, provided that the two antibodies cannot displace each other when they bind EGFR.

Methods of identifying the binding epitopes of antibodies are well known in the art. Briefly, antibody binding epitopes can be determined by an antibody displacement assay. This may provide an initial understanding to the binding site. Antibody displacement techniques are well known in the art and described in length in WO2010/029534, incorporated herein by reference.

Once the binding properties of the antibodies are characterized, a combination of at least two antibodies of the anti EGFR antibodies exhibiting binding to different epitopes on the EGFR (as described hereinabove), is selected.

Those antibody combinations selected according to the present teachings can be further qualified having anti tumor activity using in vitro and in vivo methods which are well known in the art and described at length in the Examples section which follows.

While implementing the present teachings, the present inventors have successfully uncovered combinations of antibodies which are most effective in treating cancer in a subject in need thereof. Such combinations include, but are not limited to:
(i) an anti-EGFR antibody comprising the CDR sequences of anti EGFR 565 (CNCM Deposit Number 1-4262); and an anti-EGFR antibody comprising the CDR sequences of C225/Cetuximab™;
(ii) an anti-EGFR antibody comprising the CDR sequences of anti EGFR 111 (CNCM Deposit Number I-4261); and an anti-EGFR antibody comprising the CDR sequences of C225/Cetuximab™;
(iii) an anti-EGFR antibody comprising the CDR sequences of anti EGFR 565 (CNCM Deposit Number 1-4262); and an anti-EGFR antibody comprising the CDR sequences of panitumumab™;
(iv) an anti-EGFR antibody comprising the CDR sequences of anti EGFR 111 (CNCM Deposit Number 1-4261); and an anti-EGFR antibody comprising the CDR sequences of panitumumab™.

The antibodies of the disclosed combination may be packaged separately.

Thus, according to another aspect of the present invention there is provided an article of manufacture comprising a packaging material identified for treating cancer, packaging:
(i) an anti-EGFR antibody comprising the CDR sequences of anti EGFR 565 (CNCM Deposit Number 1-4262); and an anti-EGFR antibody comprising the CDR sequences of C225/Cetuximab™; or
(ii) an anti-EGFR antibody comprising the CDR sequences of anti EGFR 111 (CNCM Deposit Number I-4261); and an anti-EGFR antibody comprising the CDR sequences of C225/Cetuximab™; or
(iii) an anti-EGFR antibody comprising the CDR sequences of anti EGFR 565 (CNCM Deposit Number 1-4262); and an anti-EGFR antibody comprising the CDR sequences of panitumumab™; or
(iv) an anti-EGFR antibody comprising the CDR sequences of anti EGFR 111 (CNCM Deposit Number 1-4261); and an anti-EGFR antibody comprising the CDR sequences of panitumumab™.

Alternatively, the antibodies of the disclosed combinations may be formulated in a single formulation.

Thus, according to another aspect of the present invention there is provided a pharmaceutical composition comprising active ingredients selected from the group consisting of:
(i) an anti-EGFR antibody comprising the CDR sequences of anti EGFR 565 (CNCM Deposit Number 1-4262); and an anti-EGFR antibody comprising the CDR sequences of C225/Cetuximab™; or
(ii) an anti-EGFR antibody comprising the CDR sequences of anti EGFR 111 (CNCM Deposit Number I-4261); and an anti-EGFR antibody comprising the CDR sequences of C225/Cetuximab™; or
(iii) an anti-EGFR antibody comprising the CDR sequences of anti EGFR 565 (CNCM Deposit Number 1-4262); and an anti-EGFR antibody comprising the CDR sequences of panitumumab™; or
(iv) an anti-EGFR antibody comprising the CDR sequences of anti EGFR 111 (CNCM Deposit Number I-4261); and an anti-EGFR antibody comprising the CDR sequences of panitumumab™,
and a pharmaceutically acceptable carrier or diluent.

As used herein the term "subject" refers to a mammal, preferably a human subject.

As used herein the term "treating" refers to alleviating or diminishing a symptom associated with a disease (e.g., cancerous disease). Preferably, treating means cures, e.g., substantially eliminates, the symptoms associated with the disease.

As used herein the term "cancer" refers to a tumoral disease which depends on EGFR (activity and/or expression) for onset and/or progression. Examples of cancer which can be treated in accordance with the present teachings include, but are not limited to, carcinoma, adenocarcinoma, lung cancer, liver cancer, colorectal cancer, brain, head and neck cancer (e.g., neuro/glioblastoma), breast cancer, ovarian cancer, transitional cell carcinoma of the bladder, prostate cancer, oral squamous cell carcinoma, bone sarcoma, biliary tract cancer such as gallbladder carcinoma (GBC), kidney cancer and pancreatic cancer.

According to a particular embodiment, the cancer is triple negative breast cancer.

Antibodies of the present invention can be administered to an organism *per se,* or in a pharmaceutical composition where they are mixed with suitable carriers or excipients (either individually or in a co-formulation).

As used herein, a "pharmaceutical composition" refers to a preparation of one or more of the active ingredients described herein with other chemical components such as physiologically suitable carriers and excipients. The purpose of a pharmaceutical composition is to facilitate administration of a compound to an organism.

As used herein, the term "active ingredient" refers to the antibodies accountable for the intended biological effect.

Hereinafter, the phrases "physiologically acceptable carrier" and "pharmaceutically acceptable carrier," which may be used interchangeably, refer to a carrier or a diluent that does not cause significant irritation to an organism and does not abrogate the biological activity and properties of the administered compound. An adjuvant is included under these phrases.

Herein, the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils, and polyethylene glycols.

Techniques for formulation and administration of drugs may be found in the latest edition of "Remington's Pharmaceutical Sciences," Mack Publishing Co., Easton, PA, which is herein fully incorporated by reference.

Suitable routes of administration may, for example, include oral, rectal, transmucosal, especially transnasal, intestinal, or parenteral delivery, including intramuscular, subcutaneous, and intramedullary injections, as well as intrathecal, direct intraventricular, intravenous, intraperitoneal, intranasal, or intraocular injections.

Alternately, one may administer the pharmaceutical composition in a local rather than systemic manner, for example, via injection of the pharmaceutical composition directly into a tissue region of a patient.

Pharmaceutical compositions of the present invention may be manufactured by processes well known in the art, e.g., by means of conventional mixing, dissolving, granulating, dragee-making, levigating, emulsifying, encapsulating, entrapping, or lyophilizing processes.

Pharmaceutical compositions for use in accordance with the present invention thus may be formulated in conventional manner using one or more physiologically acceptable carriers comprising excipients and auxiliaries, which facilitate processing of the active ingredients into preparations that can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

For oral administration, the pharmaceutical composition can be formulated readily by combining the active compounds with pharmaceutically acceptable carriers well known in the art. Such carriers enable the pharmaceutical composition to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries as desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, and sodium carbomethylcellulose; and/or physiologically acceptable polymers such as polyvinylpyrrolidone (PVP). If desired, disintegrating agents, such as cross-linked polyvinyl pyrrolidone, agar, or alginic acid or a salt thereof, such as sodium alginate, may be added.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions, and suitable organic solvents or solvent mixtures. Dyestuffs or pigments may be added to the tablets or dragee coatings for identification or to characterize different combinations of active compound doses.

Pharmaceutical compositions that can be used orally include push-fit capsules made of gelatin, as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate, and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

For administration by nasal inhalation, the active ingredients for use according to the present invention are conveniently delivered in the form of an aerosol spray presentation from a pressurized pack or a nebulizer with the use of a suitable propellant, e.g., dichlorodifluoromethane, trichlorofluoromethane, dichloro-tetrafluoroethane, or carbon dioxide. In the case of a pressurized aerosol, the dosage may be determined by providing a valve to deliver a metered amount. Capsules and cartridges of, for example, gelatin for use in a dispenser may be formulated containing a powder mix of the compound and a suitable powder base, such as lactose or starch.

The pharmaceutical composition described herein may be formulated for parenteral administration, e.g., by bolus injection or continuous infusion. Formulations for injection may be presented in unit dosage form, e.g., in ampoules or in multidose containers with, optionally, an added preservative. The compositions may be suspensions, solutions, or emulsions in oily or aqueous vehicles, and may contain formulatory agents such as suspending, stabilizing, and/or dispersing agents.

Pharmaceutical compositions for parenteral administration include aqueous solutions of the active preparation in water-soluble form. Additionally, suspensions of the active ingredients may be prepared as appropriate oily or water-based injection suspensions. Suitable lipophilic solvents or vehicles include fatty oils such as sesame oil, or synthetic fatty acid esters such as ethyl oleate, triglycerides, or liposomes. Aqueous injection suspensions may contain substances that increase the viscosity of the suspension, such as sodium carboxymethyl cellulose, sorbitol, or dextran. Optionally, the suspension may also contain suitable stabilizers or agents that increase the solubility of the active ingredients, to allow for the preparation of highly concentrated solutions.

Alternatively, the active ingredient may be in powder form for constitution with a suitable vehicle, e.g., a sterile, pyrogen-free, water-based solution, before use.

The pharmaceutical composition of the present invention may also be formulated in rectal compositions such as suppositories or retention enemas, using, for example, conventional suppository bases such as cocoa butter or other glycerides.

Pharmaceutical compositions suitable for use in the context of the present invention include compositions wherein the active ingredients are contained in an amount effective to achieve the intended purpose. More specifically, a "therapeutically effective amount" means an amount of active ingredients (e.g., a nucleic acid construct) effective to prevent, alleviate, or ameliorate symptoms of a disorder (e.g., ischemia) or prolong the survival of the subject being treated.

Determination of a therapeutically effective amount is well within the capability of those skilled in the art, especially in light of the detailed disclosure provided herein.

For any preparation used in the methods of the invention, the dosage or the therapeutically effective amount can be estimated initially from in vitro and cell culture assays. For example, a dose can be formulated in animal models to achieve a desired concentration or titer. Such information can be used to more accurately determine useful doses in humans.

Since administration of the antibody combination is expected to produce improved results over the administration of single antibodies, the therapeutically effective dose of each of the antibodies in the combined treatment may be for example less than 50 %, 40 %, 30 % 20 % or even less than 10 % the of the FDA approved dose.

Toxicity and therapeutic efficacy of the active ingredients described herein can be determined by standard pharmaceutical procedures in vitro, in cell cultures or experimental animals. The data obtained from these in vitro and cell culture assays and animal studies can be used in formulating a range of dosage for use in human. The dosage may vary depending upon the dosage form employed and the route of administration utilized. The exact formulation, route of administration, and dosage can be chosen by the individual physician in view of the patient's condition. (See, e.g., Fingl, E. et al. (1975), "The Pharmacological Basis of Therapeutics," Ch. 1, p.1).

Dosage amount and administration intervals may be adjusted individually to provide sufficient plasma or intrathecal levels of the active ingredient to induce or suppress the biological effect (i.e., minimally effective concentration, MEC). The MEC will vary for each preparation, but can be estimated from in vitro data. Dosages necessary to achieve the MEC will depend on individual characteristics and route of administration. Detection assays can be used to determine plasma concentrations.

Depending on the severity and responsiveness of the condition to be treated, dosing can be of a single or a plurality of administrations, with course of treatment lasting from several days to several weeks, or until cure is effected or diminution of the disease state is achieved.

The amount of a composition to be administered will, of course, be dependent on the subject being treated, the severity of the affliction, the manner of administration, the judgment of the prescribing physician, etc.

Typically used models for analyzing the effect of the agents described herein on tumors are provided infra.

An animal model for triple negative breast cancer is Herschkowitz et al., Breast Dis. 2010;32(1-2):63-71; Kaur et al. BMC Cancer 2012, 12:120.

Compositions of the present invention may, if desired, be presented in a pack or dispenser device, such as an FDA-approved kit, which may contain one or more unit dosage forms containing the active ingredient. The pack may, for example, comprise metal or plastic foil, such as a blister pack. The pack or dispenser device may be accompanied by instructions for administration. The pack or dispenser device may also be accompanied by a notice in a form prescribed by a governmental agency regulating the manufacture, use, or sale of pharmaceuticals, which notice is reflective of approval by the agency of the form of the compositions for human or veterinary administration. Such notice, for example, may include labeling approved by the U.S. Food and Drug Administration for prescription drugs or of an approved product insert. Compositions comprising a preparation of the invention formulated in a pharmaceutically acceptable carrier may also be prepared, placed in an appropriate container, and labeled for treatment of an indicated condition, as further detailed above.

It will be appreciated that the antibodies of the present invention can be provided to the individual with additional active agents to achieve an improved therapeutic effect as compared to treatment with the antibodies alone. In such therapy, measures (e.g., dosing and selection of the complementary agent) are taken to adverse side effects which may be associated with combination therapies.

Administration of such combination therapy can be simultaneous, such as in a single capsule having a fixed ration of these active agents, or in multiple capsules for each agent.

Thus, for example, the antibodies of the present invention can be administered along with analgesics, chemotherapeutic agents (e.g., anthracyclins), radiotherapeutic agents, hormonal therapy and other treatment regimens which are well known in the art.

It is expected that during the life of a patent maturing from this application many relevant antibodies will be developed and the scope of the term of the patent is intended to include all such new technologies *a priori.*

The terms "comprises", "comprising", "includes", "including", "having" and their conjugates mean "including but not limited to". This term encompasses the terms "consisting of" and "consisting essentially of".

The phrase "consisting essentially of" means that the composition or method may include additional ingredients and/or steps, but only if the additional ingredients and/or steps do not materially alter the basic and novel characteristics of the claimed composition or method.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader. All the information contained therein is incorporated herein by reference.

### GENERAL MATERIALS AND METHODS

***Reagents and antibodies:*** Unless indicated, materials were from Sigma (St Louis, MO) and antibodies from Santa Cruz Biotechnology (Santa Cruz, CA). Other antibodies were from Covance (Princeton, NJ; anti-ubiquitin), Alexis Biochemicals (San Diego, CA; anti-EGFR), and Cell Signaling (Beverly, MA; anti-pAKT, anti-pEGFR, and Rab-11). mAbs 565 and 111 were generated in our laboratories (11). siRNA oligonucleotides were purchased from Dharmacon Thermo Fisher Scientific (Lafayette, CO).

***Antibody radiolabeling and displacement assays:*** Panitumumab was radiolabeled by using Na¹²⁵I (0.5 mCi) and chloramine T. The reaction mixture was chromatographed on Sephadex G-25, yielding specific activity of 1-2 mCi/mg protein. For displacement assays, cells (250,000/well) were grown in 24-well plates and treated for 60 min at 4°C with increasing concentrations of unlabeled mAbs. Thereafter, the radiolabeled mAb (8 nmol/L) was added, and cells were incubated for an additional 30 min. After washing, cells were solubilized and their radioactivity determined. Antibody competition assays used cells that were plated in 96-well plates (10,000 cells/well). On the next day, cells were incubated at 4°C for 15 min with increasing concentrations of competitors in KREB buffer (146 mM NaCl, 4 mM KCl, 0.5 mM MgCl₂, 1 mM CaCl₂, 10 mM HEPES, 1 gr/L glucose and 1 mg/ml albumin), and then with mAbs for additional 45 min. After washing, cells were incubated for 60 min with an HRP-conjugated anti-human antibody and then 2-2' Azino-bis (3-ethylbenzothiazoline-6-sulfonic acid) was added. The optical density was determined using a plate reader (415 nm).

***Flow cytometry and immunofluorescence:*** Following incubation with EGF or mAbs, cells were trypsinized and incubated at 4°C. Thereafter, mAbs were stripped from the cell surface with an acidic solution (0.2 M Na-acetate, 0.5 M NaCl), and cells were incubated with primary and secondary (fluorescent) antibodies at 4°C. Finally, surface EGFRs were quantified using cytofluorimetry. Prior to immunofluorescence analysis, cells were fixed, permeabilized (0.1% Triton X-100) and blocked with albumin (1 mg/ml). Cells were incubated overnight with primary antibodies at 4°C, and with secondary fluorescent antibodies for 1 hour at room temperature. Imaging was performed using a confocal Zeiss LSM 710 microscope.

***Cell lines and transfections:*** Cell lines were obtained from the American Type Culture Collection (Manassas, VA) and grown in RPMI or Dulbecco's modified Eagle's medium, supplemented with fetal calf serum (10 % vol/vol) and 1 mM sodium pyruvate. For transient plasmid and siRNA transfections JetPEI (Polyplus, Illkirch, France) was used and either Dharmafect from Dharmacon or HiPerfect from Qiagen (Hilden, Germany), respectively.

***EGF displacement assays using ELISA:*** Cells were plated in 96-well plates and on the next day they were incubated for 45 minutes at 4 °C with increasing concentrations of mAbs in KREB buffer. Afterwards, EGF-Texas Red was added for an additional 45 minutes. After washing, fluorescent signals were determined using a microplate reader.

***Invasion and migration assays:*** BT549 cells (10 x 10⁵/well) were seeded in the upper compartment of Transwell (Corning) or Matrigel-coated invasion chambers. The lower compartment was filled with serum-containing medium. Eighteen hours later, cells on the lower side of the filter were fixed, permeabilized with Triton X-100 (0.1%), and stained with crystal violet. Images were quantified by using ImageJ.

***Tumorigenic growth in animals:*** CD/nude mice were divided in groups of 5 mice and injected subcutaneously with HCC70 cells (5x10⁶ per mouse). mAbs were injected intraperitoneally at 160 µg/mouse/injection on days 7, 14, 21, 28 and 35 after grafting. Tumor volume was evaluated once per week.

***ImageStream analysis:*** Following treatments, cells were trypsinized, fixed, permeabilized and stained with an anti-EGFR antibody and a secondary anti-rabbit antibody. 10,000 events from each sample were collected using the ImageStreamX instrument (Amnis corp., Seattle, WA) and the IDEAS 4.0 software. Single cells were gated using the area and aspect ratio features, as well as the Gradient RMS feature (26). Cells were then gated to select only positively stained cells using their pixel intensity values. The localization of EGFR was calculated using the Max Contour Position feature.

***Statistical analysis:*** Two way ANOVA and Bonferroni multiple comparison tests were used to analyze differences between groups. P values < 0.01 were considered significant.

### EXAMPLE 1

### Certain combinations of anti-EGFR mAbs enhance receptor downregulation

To identify pairs of anti-EGFR mAbs which work synergistically, MDA-MB-468 TNBC cells were incubated with a fluorescent derivative of EGF, in the presence of anti-EGFR mAbs 111, 565, panitumumab and cetuximab. Three of the four antibodies effectively displaced EGF, but mAb565 behaved as a relatively weak competitor (Figure 1A). Next, panitumumab was radiolabeled. It was found that the two clinically approved antibodies share antigenic epitopes, therefore they may not synergize, but mAb565 engages a distinct epitope of EGFR (Figures 7A-B). Similar analyses revealed that mAb111 binds EGFR independently from the binding of cetuximab and panitumumab (Figures 1B and 1C), and according to previous report mAbs 111 and 565 are non-competitive (11). These lines of evidence excluded the panitumumab plus cetuximab pair, and identified several combinations (e.g., 111/panitumumab and 111/cetuximab) as potential collaborative pairs. A standard EGFR downregulation assay, performed with HeLa cells, validated these predictions (Figure ID): long-term exposure to the non-competitive mAb combinations achieved receptor downregulation. This was also confirmed by immunoblot analyses, showing that receptor down-regulation closely reflected degradation (Figure IE). Interestingly, mAb565 induced more extensive downregulation and degradation of EGF, an activity that may be attributed to a faint ability to stimulate EGFR auto-phosphorylation (Figure IE). Similarly, exposure of HeLa cells to either mAb111 or panitumumab induced weak auto-phosphorylation, but their combination strongly induced both phosphorylation and degradation (Figure IF). In conclusion, the ability of mAbs to downregulate EGFR and promote degradation correlates with the engagement of non-overlapping sites, and it might relate to stimulation of receptor auto-phosphorylation.

### EXAMPLE 2

### When combined, non-competitive mAbs induce EGFR ubiquitination

Because EGF-induced ubiquitination of EGFR by c-CBL is tightly associated with receptor degradation (17), receptor ubiquitination following treatment with the panitumumab plus mAb111 pair was tested. Unlike single mAbs, the combined treatment increased EGFR ubiquitination (Figure 2A). As expected, EGF-induced ubiquitination appeared earlier and, interestingly, yielded a mixture of higher molecular weight species. Another difference emerged from a co-immunoprecipitation experiment: Unlike the well-characterized EGF-induced formation of EGFR-CBL complexes, neither a single mAb nor a combination resulted in detectable complexes (Figure 2B). Nevertheless, both EGF- and mAb-induced EGFR degradation were inhibited by a blocker of lysosomal hydrolases (bafilomycin), but an antagonist of the 26S proteasome (bortezomib) was ineffective (Figure 2C). Conceivably, mAb mixtures accelerate lysosomal degradation by inducing relatively slow ubiquitination of EGFR and recruiting an E3 ligase distinct from c-CBL.

The wealth of information on EGF-induced receptor degradation contrasts with the paucity of data on mAb-induced endocytosis. Hence, a library of siRNAs arranged in 180 pools, each targeting a single endocytic mediator was screened (see Methods). HeLa cells were transfected with individual pools, along with control siRNAs. Thereafter, cells were treated with a mAb mixture and incubated for 12 hours, prior to analysis of EGFR degradation. A validation test, and a list, of positive hits (in two cycles) are depicted in Figure 8. Remarkably, two proteins that play critical roles in clathrin-mediated endocytosis, Huntingtin Interacting Protein 1 (HIP1) and dynamin 2 (DNM2), a GTPase, scored positively. In addition to the dynamin's partner, amphiphysin, and the ubiquitin binder EPS15L1, the screen identified several proteins involved in late stages of the endocytic pathway, such as RAB7L1 (18) and TSG101, an E2-like molecule necessary for cargo sorting. These observations are consistent with a model assuming that mAb combinations divert EGFR from the recycling route to late endosomes and lysosomes (12).

### EXAMPLE 3

### Antibody-induced endocytosis of EGFR avoids the recycling compartment

To examine a scenario of mAb-mediated inhibition of recycling, subcellular distribution of EGFR was analyzed. As predicted, treatment with a mAb mixture translocated EGFR of HeLa cells into intracellular vesicles (Figure 9). While EGF induced a similar translocation, treatment with single mAbs was ineffective. These differences were quantified using flow cytometry (Figures 3A and 3B): incubation with either EGF or two mAbs increased the intracellular fraction. To directly follow the recycling route, the present inventors employed an antibody to RAB11, a GTPase of recycling endosomes (18). As expected, in untreated cells EGFR and RAB11 displayed non-overlapping patterns, but EGF translocated the receptor to the perinuclear recycling compartment (Figure 3C). Importantly, although the mAb mixture extensively translocated EGFR into intracellular puncta, these structures were devoid of RAB11, in line with the possibility that mAb mixtures sort EGFR for degradation while avoiding the recycling route.

### EXAMPLE 4

### mAb-induced degradation of EGFR is independent of the kinase activity and the intrinsic dimerization ability of EGFR

Due to their ability to force EGFR dimerization, antibodies might act as partial agonists. To examine this extracts of stimulated HeLa cells were probed. As expected, EGF enhanced phosphorylation of EGFR, as well as phosphorylation of two effectors, AKT and ERK (Figure 4A). Remarkably, although the agonist antibody 565 and mixtures of non-competitive mAbs enhanced EGFR auto-phosphorylation, as well as accelerated receptor degradation, this did not translate to ERK or AKT activation. Hence, it was assumed that mAb-mediated and EGF-induced dimerization remarkably differ. Next, a previously described EGFR mutant, which is defective in both homodimer formation and EGF-induced internalization (19) was employed. Using this deletion mutant (ΔCR1), the present inventors confirmed that dimerization is essential for both EGF-induced auto-phosphorylation and degradation of EGFR (Figure 4B). Strikingly, neither mAb-mediated auto-phosphorylation nor mAb-mediated degradation of EGFR displayed dependence on the intrinsic dimer forming ability of EGFR, implying that mAb-induced clustering preempts the intrinsic dimer-forming feature of EGFR.

The EGFR specific TKI, AG1478, was employed in an effort to clarify the contribution of the intrinsic kinase activity to mAb-induced endocytosis. Although treatment with AG1478 completely blocked both EGF and mAb-induced auto-phosphorylation, only the ligand-induced degradation of EGFR was inhibited (Figure 4C). Conceivably, EGFR is passively recruited by mAbs: neither kinase activity nor dimer forming competence are needed, but all three functions are essential for EGF-induced degradation of EGFR.

### EXAMPLE 5

### EGFR downregulation impairs the ability of TNBC cells to migrate

Node-positive TNBC is associated with higher EGFR expression compared to node-negative lesions (20), suggesting that EGFR drives tumor progression. As a prelude to testing the applicability of mAb-mediated downregulation, EGFR status was surveyed in a series of TNBC cell lines (Figure 10A). This analysis confirmed EGFR overexpression in TNBC cell lines; lines that belong to other subtypes, such as HER2⁺ (SKBR3 and T47D) and the luminal type (MCF7), displayed lower EGFR levels. Next, BT-549 TNBC cells were transfected with EGFR-specific siRNAs (Figure 10B), and found that loss of >80% of the receptor associated with 60% or 80% reduction in the ability of cells to migrate or invade through an extracellular barrier, respectively (Figures 10C-D). Next, the present inventors asked whether a similar effect would accompany mAb-induced endocytosis. Using a set of TNBC cell lines and mAb mixtures EGFR downregulation was observed (Figure 5A), similar to the effects on HeLa cells (Figure ID): when singly applied, mAbs other than the partial agonist, 565, were unable to downregulate EGFR, but mixtures of non-competitive mAbs caused EGFR degradation in all tested TNBC lines. Notably, EGF was unable to downregulate EGFR of MDA-MB468 cells, probably due to saturation of the endocytic pathway by the highly expressed receptor (21). Nevertheless, reproducible downregulation was achieved by mAbs, in line with distinct routes of endocytosis engaged by mAbs and EGF.

Next, the present inventors tested the prediction that mAb combinations would inhibit migration of TNBC cells. For this, BT-549 cells were used and a mAb combination, which effectively downregulated EGFR (Figure 5B). Under these conditions almost 50% reduction in cell migration was observed, but neither mAb, when singly applied, was effective (Figures 5C and 5D). These findings are consistent with the observations made using siRNAs specific to EGFR (Figure 10). In conclusion, EGFR appears to play an important role in the migratory behavior of the TNBC model employed, such that mAb-induced endocytosis and degradation of EGFR can significantly limit cell migration *in vitro.*

### EXAMPLE 6

### Mixtures ofmAbs arrest TNBC cells at G1 and inhibit their tumorigenic growth in animals

Prolonged exposure of HCC70 cells to cetuximab, or to cetuximab plus mAb565, exerted only minor effects on cell survival, as reflected by the unchanged sub-G1 fraction (Figure 11A). In contrast, exposure to an apoptosis-inducing drug, puromycin, increased sub-Gl and erased the S-phase fraction, consistent with up-regulation of the cleaved form of caspase-3 (Figure 11B). Although the mAbs induced no detectable apoptosis, they clearly arrested cells at G1 (Figure 11A). To measure proliferation, HCC70 cells were incubated for 5 days with another pair of non-competitive mAbs, in the absence or presence of EGF (Figure 6A). Although, EGF strongly promoted proliferation of HCC70 cells, this effect was almost completely inhibited by the combination of mAbs. Notably, each antibody exerted only a small inhibitory effect when singly applied, in line with the proposed effect of antibody-induced endocytosis of EGFR.

Animal studies using HCC70 cells examined the relevance of the present observations to tumor growth. Cells were injected subcutaneously and once tumors became palpable, the present inventors injected into the peritoneum either single mAbs or mAb combinations. Figures 6B and 6C present the results: differences emerged following 4 week of tumor growth: each mAb induced a partial inhibitory effect, but the combinations more effectively regressed tumors. Importantly, the inhibitory effects progressively increased, such that the cetuximab plus mAb111 combination reached statistical significance (p<0.01) at week 8, and the other combination clearly showed a similar trend at the time of trial termination.

In summary, although TNBC clinical trials employing EGFR inhibitors, including cetuximab, reported lack of clinical benefit (22), the present results offer an alternative strategy. This strategy combines non-competitive mAbs to achieve robust EGFR degradation. Similar to EGF, non-competitive mAbs target EGFR to degradation in lysosomes and they engage ubiquitination and the clathrin-mediated route. Nevertheless, mAb-induced degradation is unique and identifies oligoclonal mixtures as a viable alternative to the singly used therapeutic mAbs: this relatively slow process is independent of c-CBL and the intrinsic kinase activity, or dimer-forming ability, of EGFR. As a result, mAb mixtures inhibit motility of TNBC cells, as well as arrest them at G1, attributes that translate to effective inhibition of tumor growth in an animal model.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting.

### REFERENCES

1. Witsch E, Sela M, & Yarden Y (2010) Roles for growth factors in cancer progression. Physiology (Bethesda) 25(2):85-101.
2. Ciardiello F & Tortora G (2008) EGFR antagonists in cancer treatment. The New England Journal of Medicine 358(11):1160-1174.
3. Ben-Kasus T, Schechter B, Sela M, & Yarden Y (2007) Cancer therapeutic antibodies come of age: targeting minimal residual disease. Mol Oncol 1(1):42-54.
4. Drebin JA, Link VC, & Greene MI (1988) Monoclonal antibodies reactive with distinct domains of the neu oncogene-encoded p185 molecule exert synergistic anti-tumor effects in vivo. Oncogene 2(3):273-277.
5. Spiridon CI, et al. (2002) Targeting multiple Her-2 epitopes with monoclonal antibodies results in improved antigrowth activity of a human breast cancer cell line in vitro and in vivo. Clinical Cancer Research 8(6):1720-1730.
6. Kasprzyk PG, Song SU, Di Fiore PP, & King CR (1992) Therapy of an animal model of human gastric cancer using a combination of anti-erbB-2 monoclonal antibodies. Cancer Research 52(10):2771-2776.
7. Ben-Kasus T, Schechter B, Lavi S, Yarden Y, & Sela M (2009) Persistent elimination of ErbB-2/HER2-overexpressing tumors using combinations of monoclonal antibodies: relevance of receptor endocytosis. Proceedings of the National Academy of Sciences of the United States of America 106(9):3294-3299.
8. Baselga J, et al. (2012) Pertuzumab plus trastuzumab plus docetaxel for metastatic breast cancer. The New England Journal of Medicine 366(2):109-119.
9. Fan Z, Masui H, Altas I, & Mendelsohn J (1993) Blockade of epidermal growth factor receptor function by bivalent and monovalent fragments of 225 anti-epidermal growth factor receptor monoclonal antibodies. Cancer Research 53(18):4322-4328.
10. Jaramillo ML, et al. (2006) Effect of the anti-receptor ligand-blocking 225 monoclonal antibody on EGF receptor endocytosis and sorting. Experimental Cell Research 312(15):2778-2790.
11. Friedman LM, et al. (2005) Synergistic down-regulation of receptor tyrosine kinases by combinations of mAbs: implications for cancer immunotherapy. Proceedings of the National Academy of Sciences of the United States of America 102(6):1915-1920.
12. Spangler JB, et al. (2010) Combination antibody treatment down-regulates epidermal growth factor receptor by inhibiting endosomal recycling. Proceedings of the National Academy of Sciences of the United States of America 107(30):13252-13257.
13. Foulkes WD, Smith IE, & Reis-Filho JS (2010) Triple-negative breast cancer. The New England Journal of Medicine 363(20):1938-1948.
14. Reis-Filho JS, et al. (2006) EGFR amplification and lack of activating mutations in metaplastic breast carcinomas. The Journal of Pathology 209(4):445-453.
15. Hoadley KA, et al. (2007) EGFR associated expression profiles vary with breast tumor subtype. BMC genomics 8:258.
16. Nogi H, et al. (2009) EGFR as paradoxical predictor of chemosensitivity and outcome among triple-negative breast cancer. Oncol Rep 21(2):413-417.
17. Levkowitz G, et al. (1999) Ubiquitin ligase activity and tyrosine phosphorylation underlie suppression of growth factor signaling by c-Cbl/Sli-1. Molecular Cell 4(6):1029-1040.
18. Zerial M & McBride H (2001) Rab proteins as membrane organizers. Nat Rev Mol Cell Biol 2(2):107-117.
19. Wang Q, Villeneuve G, & Wang Z (2005) Control of epidermal growth factor receptor endocytosis by receptor dimerization, rather than receptor kinase activation. EMBO Rep 6(10):942-948.
20. Sutton LM, et al. (2010) Intratumoral expression level of epidermal growth factor receptor and cytokeratin 5/6 is significantly associated with nodal and distant metastases in patients with basal-like triple-negative breast carcinoma. Am J Clin Pathol 134(5):782-787.
21. Wiley HS (1988) Anomalous binding of epidermal growth factor to A431 cells is due to the effect of high receptor densities and a saturable endocytic system. The Journal of Cell Biology 107(2):801-810.
22. Burness ML, Grushko TA, & Olopade OI (2010) Epidermal growth factor receptor in triple-negative and basal-like breast cancer: promising clinical target or only a marker? Cancer J 16(1):23-32.
23. Koefoed K, et al. (2011) Rational identification of an optimal antibody mixture for targeting the epidermal growth factor receptor. mAbs 3(6):584-595.
24. Goh LK, Huang F, Kim W, Gygi S, & Sorkin A (2010) Multiple mechanisms collectively regulate clathrin-mediated endocytosis of the epidermal growth factor receptor. The Journal of Cell Biology 189(5):871-883.
25. Sigismund S, et al. (2005) Clathrin-independent endocytosis of ubiquitinated cargos. Proceedings of the National Academy of Sciences of the United States of America 102(8):2760-2765.
26. George TC, et al. (2006) Quantitative measurement of nuclear translocation events using similarity analysis of multispectral cellular images obtained in flow. Journal of Immunological Methods 311(1-2):117-129.

## Claims

1. An anti-EGFR antibody combination for use in treating pancreatic cancer, wherein the combination comprises:
(i) a first anti-EGFR antibody comprising the CDR sequences of anti EGFR 565 (CNCM Deposit Number I-4262), wherein said first anti-EGFR antibody binds the same EGFR epitope as said anti EGFR 565; and
(ii) a second anti-EGFR antibody comprising the CDR sequences of C225/Cetuximab, wherein said second anti-EGFR antibody binds the same EGFR epitope as said C225/Cetuximab.

2. The anti-EGFR antibody combination of claim 1, wherein said first anti-EGFR antibody and said second anti-EGFR are co-formulated.

3. The anti-EGFR antibody combination of claim 1, wherein said first anti-EGFR antibody and said second anti-EGFR are in separate formulations.

4. The anti-EGFR antibody combination of claim 1, wherein said first anti-EGFR antibody and said second anti-EGFR antibody are full length antibodies.
